(19) Europäisches Patentamt
European Patent Office
Office européen des brevets

(11) **EP 4 330 910 B1**

(12) **EUROPEAN PATENT SPECIFICATION**

(45) Date of publication and mention
of the grant of the patent:
**24.09.2025 Bulletin 2025/39**

(21) Application number: **22726051.0**

(22) Date of filing: **27.04.2022**

(51) International Patent Classification (IPC):
***G06T 7/00*** *(2017.01)*

(52) Cooperative Patent Classification (CPC):
**G06T 7/0016;** G06T 2207/10088; G06T 2207/30081

(86) International application number:
**PCT/EP2022/061221**

(87) International publication number:
**WO 2022/229265 (03.11.2022 Gazette 2022/44)**

(54) **SYSTEM FOR HELPING DIAGNOSING AGGRESSIVE PROSTATE CANCERS AND RELATED METHOD**

SYSTEM ZUR UNTERSTÜTZUNG BEI DER DIAGNOSE VON AGGRESSIVEM PROSTATAKREBS UND ZUGEHÖRIGES VERFAHREN

SYSTÈME D'AIDE AU DIAGNOSTIC DES CANCERS AGRESSIFS DE LA PROSTATE ET PROCÉDÉ ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2021 EP 21305545**

(43) Date of publication of application:
**06.03.2024 Bulletin 2024/10**

(73) Proprietors:
- **INSTITUT NATIONAL DE LA SANTE ET DE LA RECHERCHE MEDICALE - INSERM**
  **75013 Paris (FR)**
- **Université Grenoble Alpes**
  **38400 Saint-Martin-d'Hères (FR)**
- **Centre National de la Recherche Scientifique**
  **75016 Paris (FR)**
- **Hospices Civils de Lyon**
  **69002 Lyon (FR)**
- **Centre Léon Bérard**
  **69008 Lyon (FR)**
- **Université Claude Bernard Lyon 1**
  **69100 Villeurbanne (FR)**

(72) Inventors:
- **SOUCHON, Rémi**
  **69003 LYON (FR)**
- **JAOUEN, Tristan**
  **69424 LYON CEDEX 03 (FR)**
- **HOANG-DINH, Au**
  **HANOI (VN)**
- **GONINDARD, Christelle**
  **38058 GRENOBLE CEDEX 9 (FR)**
- **ROUVIERE, Olivier**
  **69003 LYON (FR)**

(74) Representative: **Lavoix**
**2, place d'Estienne d'Orves**
**75441 Paris Cedex 09 (FR)**

(56) References cited:
**US-A1- 2003 105 605     US-A1- 2020 015 734**
**US-A1- 2020 278 408**

- **LITJENS GEERT ET AL: "Computer-Aided Detection of Prostate Cancer in MRI", IEEE TRANSACTIONS ON MEDICAL IMAGING, IEEE SERVICE CENTER, PISCATAWAY, NJ, US, vol. 33, no. 5, May 2014 (2014-05-01), pages 1083 - 1092, XP011546108, ISSN: 0278-0062, [retrieved on 20140422], DOI: 10.1109/TMI.2014.2303821**

EP 4 330 910 B1

• GIANNINI VALENTINA ET AL: "A fully automatic computer aided diagnosis system for peripheral zone prostate cancer detection using multi-parametric magnetic resonance imaging", COMPUTERIZED MEDICAL IMAGING AND GRAPHICS, PERGAMON PRESS, NEW YORK, NY, US, vol. 46, 12 September 2015 (2015-09-12), pages 219 - 226, XP029362423, ISSN: 0895-6111, DOI: 10.1016/J.COMPMEDIMAG.2015.09.001

## Description

The present invention concerns a system for helping diagnosing aggressive prostate cancers, as well as a method for helping diagnosing aggressive prostate cancers.

With more than one million new cases each year, prostate cancer is the most prevalent cancer in men worldwide, and the second cancer-related cause of death. The diagnosis relies on ultrasound-guided biopsies. However often ultrasound does not show the tumor, and the role of ultrasound is limited to guiding the biopsy needle into the prostate.

The practitioner typically performs 12 systematic biopsies. It is not uncommon that biopsies miss clinically significant tumors. As a consequence, the patient does not receive appropriate treatment.

In other patients, the biopsy needle may accidentally fall within an isolated spot of cancerous cells, when the actual tumor is small and indolent and is not threatening the patient's health. As a consequence, these patients receive aggressive treatment, possibly with severe adverse effects such as incontinence and impotence, with no medical benefit.

In order to improve biopsy targeting, some image processing methods that try to recognize the signs of aggressive cancer in MRI images have been proposed, for example in the articles by Au Hoang Dinh et al. " Quantitative analysis of prostate multiparametric MR images for detection of aggressive prostate cancer in the peripheral zone : a multiple imager study", published in 2016 in the journal Radiology, volume 280, number 1, page 117, and "Characterization of prostate cancer with Gleason score of at least 7 by using quantitative multiparametric MR imaging : validation of a computer-aided diagnosis system in patients referred for prostate biopsy", published in 2018 in the journal Radiology, volume 287, number 2, page 525.

However, the known methods remain less reliable for detecting aggressive cancers than an experienced radiologist would be. In addition, these pre-existing methods have usually been developed using a base of MR images acquired by a limited number of MR scanners, and their reliability decreases significantly when used to detect cancers on MR images acquired using a different type of MR scanner than those used in the original base.

There is therefore a need for a system, able to help a radiologist diagnose aggressive prostate cancers, that reliably detects aggressive prostate cancers while being more robust than the pre-existing systems, and notably able to be used reliably with MR images that were acquired using different types of MR scanners.

In this view, the present specification concerns a system for helping diagnosing aggressive prostate cancers according to claim 1.

According to specific embodiments, the system is according to any one of claims 2 to 11.

The present specification also concerns a method for helping diagnosing aggressive prostate cancers according to claim 12.

The present specification also concerns a computer program product according to claim 13.

The present specification also concerns an information medium according to claim 14.

The present specification further concerns a method for diagnosing aggressive prostate cancers, comprising a step for implementing a method according to claim 12.

Features and advantages of the invention will appear upon reading the following specification, given only as a non-limiting example, and made with reference to the associated drawings, in which :

- Figure 1 is a schematics of a system for helping diagnose aggressive prostate cancers, and
- Figure 2 is a flow chart of the steps of an example of a method for helping diagnose aggressive prostate cancers, implemented by the system of figure 1,
- Figure 3 is a graph of the specificity of an example of system according to figure 1, as a function of several parameters,
- Figure 4 is a graph of the specificity of another example of system according to figure 1, as a function of several parameters, and
- Figure 5 is a graph of the specificity of yet another example of system according to figure 1, as a function of several parameters.

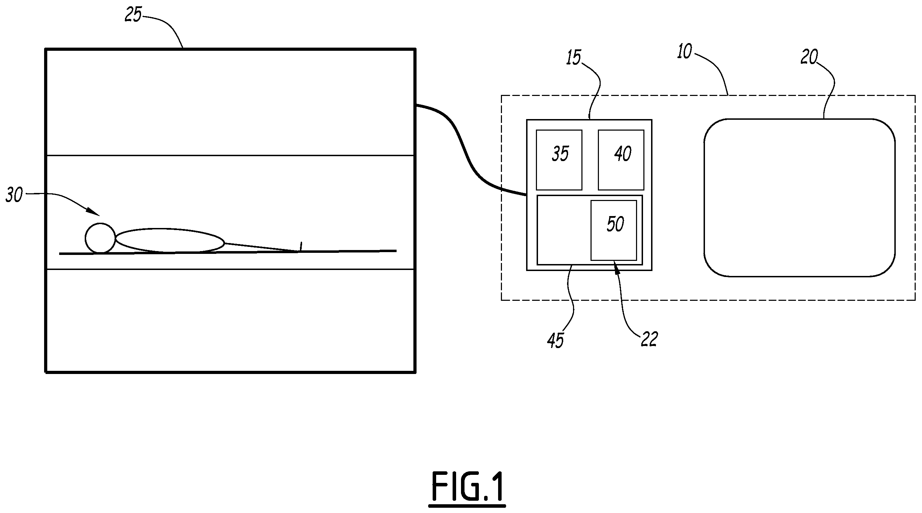

A schematics of a system 10 for helping diagnose aggressive prostate cancers is shown on figure 1.

The system 10 comprises a control module 15, a human-machine interface 20 and a computer program product 22.

More generally, the system 10 is an electronic computer capable of handling and/or transforming data illustrated as electronic or physical amounts in registers of the system 10 and/or memories in other similar data corresponding to physical data in the memories, registers or other types of display, transmission or memory storage devices.

The system 10 is configured to implement a method for helping aggressive prostate cancer diagnosis.

The control module 15 is configured to receive, from a distinct device 25 such as a magnetic resonance imagery scanner 25, at least one magnetic resonance image of a subject 30's prostate.

The control module 15 comprises, for example, a data processing unit, such as a processor 35, a memory 40 and a reader 45 of a legible information medium 50.

The computer program product 22 includes the information medium 50.

A legible information medium 50 is a medium legible by the data processing unit 35. The legible information medium 50 is a medium suitable for storing in memory electronic instructions and capable of being coupled with a bus of a computer system.

As an example, the legible information medium 50 is an optical disc, a CD-ROM, a magneto-optical disc, a ROM memory, a RAM memory, an EPROM memory, an EEPROM memory, a magnetic card or an optical card.

On the legible information medium 50, a program comprising program instructions is stored in memory.

The computer program may be loaded on the data processing unit 35 and is adapted for causing the application of a method for helping diagnose prostate aggressive cancers when the computer program is applied on the processor 35.

The human-machine interface 20 is configured to enable transmission of information between a human, such as a physician operating the system 10, and the control module 15.

The human-machine interface 20 comprises a display screen, for example a touch screen. Optionally, the human-machine interface 20 further comprises a mouse and/or a keyboard and/or a loudspeaker and/or a microphone.

The scanner 25 is, for example, distinct from the system 10, in particular disposed in a different room or a different building, and linked to the system 10 by a wire or wireless network.

In a possible variant, the scanner 25 is part of the system 10. For example, the control module is able to command the scanner 25 to acquire the magnetic resonance image(s).

In another variant, the distinct device 25 is a server or a database in which the magnetic resonance image(s) are stored, the magnetic resonance image(s) being in this case, for example, acquired by one or several scanner(s) and transmitted to the distinct device 25 through the network.

The scanner is a multiparametric magnetic resonance imaging ("MRI") scanner, able to acquire images of a subject's organ using different MRI methods, as will appear below, notably by measuring the T1-weighed and T2-weighed MRI signals emitted by the organ.

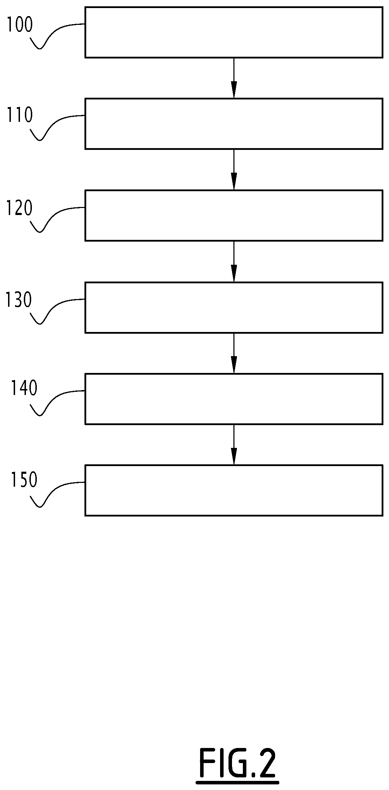

The operation of the system 10 in interaction with the computer program product is now described with reference to figure 2, which illustrates an exemplary application of a method for helping diagnose aggressive prostate cancers.

The method for helping diagnose aggressive prostate cancers comprises an acquisition step 100, a transmission step 110, a selection step 120, a calculation step 130, a determination step 140, and a signalling step 150.

During the acquisition step 100, at least one magnetic resonance image is acquired by the scanner 25, for example a set of magnetic resonance images.

Each image is an image of a subject's prostate.

The subject is, for example, a patient showing symptoms that are likely to be caused by a prostate cancer. In a variant, the subject does not show any symptom.

Each image is an image of at least part of the peripheral zone ("PZ") of the subject's prostate. In particular, each image is an image of at least part of the PZ and of at least part of the transition zone ("TZ") of the prostate.

The set of magnetic resonance image(s) comprises at least one first image and optionally one second image or several second image(s).

Each image comprises a set of picture elements or "pixels".

The or each first image is, for example, an apparent diffusion coefficient mapping. Such a mapping is a two-dimension representation of the values of the apparent diffusion coefficient of the imaged areas of the prostate. In such a mapping, each pixel comprises a value of the apparent diffusion coefficient of a corresponding area of the prostate.

The apparent diffusion coefficient is a measure of the magnitude of diffusion of water molecules within tissue, and is obtained through diffusion-weighted imaging (DWI). Apparent diffusion coefficient values are calculated, in a *per se* known manner, from an initial T2*-weighed image of the prostate and a set of at least two diffusion-weighed images, each diffusion-weighed image corresponding to a spatial direction and being a mapping of the T2*-signal, attenuated as a function of how easily water can diffuse in the corresponding direction.

The apparent diffusion coefficient is expressed square millimetre per second ($mm^2/s$).

At least one second image is, for example, a T1-weighed image comprising a set of pixels, each pixel being associated with a value of an intensity of a T1-weighed signal originating from the corresponding area of the prostate. In particular, a set of successive T1-weighed images are acquired during a time period.

The time period is a time period during which a contrast agent enters or leaves the prostate. In other words, the volumic concentration of the contrast agent increases monotonically or decreases monotonically during the time period.

For example, the contrast agent is injected to the subject, and the concentration of contrast agent increases, in the prostate and notably in each first or second area, until attaining a maximum value, and later decreases as the contrast agent is evacuated from the prostate. The T1-weighed signal intensity of each area of the prostate is a function of the contrast agent concentration, and notably increases as a function of the contrast agent concentration, although this increase is non linear. The T1-weighed signal intensity thus follows an increasing then decreasing trend, like the contrast agent concentration, over time.

The images are acquired during a time period following the injection, during which period the contrast agent enters the prostate. In a variant, the images are acquired during a time period following a moment of maximum concentration of contrast agent in the prostate, during which period the contrast agent is evacuated from the prostate after reaching its maximum concentration.

The contrast agent is, for example, gadolinium.

During the transmission step 110, all first or second images are transmitted to the control module 15.

For example, all the first or second images are acquired by the scanner 25 and immediately transmitted to the control module 15 through the network.

In a possible variant, the first or second images are sent, by the scanner 25, to a database or a server, are stored on the database or the server for some time, and are transmitted to the control module 15 after the storage period has expired, for example when a physician decides to ascertain whether or not the subject-prostate contains an aggressive cancer. In this case, the method for helping diagnose aggressive prostate cancers may be considered to not comprise the acquisition step 100.

During the selection step 120, at least one first portion of the PZ is selected.

For example, at least one image, such as the or one of the first image(s) is presented to a physician on the human-machine interface 20, and the physician delineates a part of the first image that the physician considers to be of interest, notably a part of the PZ that the physician considers to possibly contain an aggressive cancer in view of the image.

Optionally, a set of different first portions of the PZ are selected, for example by the physician delineating a plurality of areas of the first image.

In a possible variant, one image such as the or one of the first image(s) is automatically divided in a plurality of portions by the control module 15, for example by superimposing a square grid onto the image, and the control module 15 then considers each of the areas of the PZ that are delimited from each other by the grid to be one such first portion.

Optionally, at least one second portion of the prostate is selected. Each second portion is a portion of the TZ.

Each second portion is, for example, delineated by a physician or selected automatically in the same way as the first portion(s).

During the calculation step 130, the control module 15 calculates a first score P based on at least the first image(s) received. Optionally, as will be detailed below, the control module further calculates a second score Y from at least the first image(s) received.

In particular, a first score P is calculated for each first portion selected, and/or a second score Y is calculated for each second portion selected.

Each first score P is a function of at least one of a first quantity $x_1$, a second quantity $x_2$ and a third quantity $x_3$.

The first quantity $x_1$ is calculated, for the first portion considered, according to the equation:

$$x_1 = ADC + a_1 \times W, \text{ (equation 1)}$$

wherein :

- $a_1$ is a first constant,
- ADC is a percentile of the apparent diffusion coefficient values of the first portion, expressed in mm²/s, and
- W is a value of a normalized wash-in rate or a value of a normalized wash-out rate of the first portion, expressed in Hertz (1 Hz = 1 s⁻¹).

W is estimated from the second images.

The parameter W is for example calculated by the control module 15 by:

- Calculating a wash-in or wash-out rate for each area of the first portion, each area corresponding to a same pixel in successive second images, and
- Calculating W as a means, notably an arithmetic means, of the wash-in or wash-out rates of the pixels corresponding to the first portion.

The normalized wash-in rate of each area is calculated by, in a known manner, generating, from the intensity values of the pixels corresponding to the area in successive second images, a curve of the intensity of the area's a T1-weighed signal as a function of time, and estimating the slope of the curve.

The normalized wash-in or wash-out rate is, notably, estimated from a normalized T1-weighted signal, where the intensity of the T1-weighed signal of a pixel at a given time is divided by the intensity of the T1-weighed signal of the same pixel before the contrast agent enters the prostate, for example before injection of the contrast agent or before the contrast agent has reached the prostate (i.e before the start of the intensity increase).

It should be noted that different methods of normalization may be used, and are equivalent to each other since the corresponding values of parameter $a_1$ and of the wash-in or cash-our rates corresponding to one method of normalization can be deduced from the values corresponding to another normalization.

In a possible variant, rather than dividing all values of the T1-weighted signal by the value of this signal before injection of the contrast agent, the wash-in or wash-out rate is calculated from the slope as expressed in % of the original value.

In such a variant, $a_1$ could be expressed in $mm^2$/percent rather that in $mm^2$, and the values of $a_1$ shown 4 would therefore be 100 times smaller than if they were expressed in $mm^2$.

It should be considered that all types of normalization of the wash-in or wash-out rates may be used indifferently when implementing the present invention, with the corresponding adaptations to the values of $a_1$ and of the unit used to express the wash-in or wash-out rate.

A slope, notably the maximum slope, of the increasing portion of the curve (corresponding to the progressive increase of contrast agent concentration in the area considered) is the area's normalized wash-in rate. In a possible variant, the area's normalized wash-in rate is the average slope of the increasing portion of the curve.

The wash-in or wash-out rate is called "normalized" wash-in or "normalized" wash-out when the intensity curve is made of normalized intensity values, i.e of values of the intensity of the T1-weighed signal of one pixel, or of an area of the second images, at different times, divided by an intensity of the T1-weighed signal of the same pixel or area before the contrast agent has been injected. Such a normalization enables easy comparison between wash-in or wash-out rates obtained from different MRI machines.

It should be noted that, as mentioned below, other methods of normalization can be used.

A slope, such as an average slope or a maximum slope, of the decreasing portion of the curve (corresponding to the progressive decrease of contrast agent concentration in the area considered after reaching a maximum) is the area's normalized wash-out rate. In practice, the normalized wash-out rate is often the average slope.

If the parameter W is a normalized wash-in rate, the parameter W is, for example, the arithmetic means of the normalized wash-in rates of the areas corresponding to each pixel of the first portion.

If the parameter W is a normalized wash-out rate, the parameter W is, for example, the arithmetic means of the normalized wash-out rates of the areas corresponding to each pixel of the first portion.

In a variant, the normalized wash-in or normalized wash-out rate is calculated by calculating, for each first portion of each second image, a mean value of the T1-weighed signals of all pixels within the first portion and by generating a normalized curve of the means values of the first portion calculated as a function of time. The generated time curve has the same overall shape as those of each pixel, since the means contrast agent concentration in the first portion considered increases and then decreases over time. In this case, the wash-in rate value is a slope, notably a maximum or average slope, of the increasing part of the generated time curve, and the normalized wash-out rate is a slope, for example a maximum slope or an average slope, of the decreasing part of the curve.

It should be noted that normalized wash-in or wash-out rates may be calculated for the second portion(s) in a similar manner as described above.

The parameter ADC is calculated by the control module 15 by arranging in increasing order the apparent diffusion coefficient values of the pixels corresponding to the first portion considered, the parameter ADC being a percentile of the apparent diffusion coefficient values of the first portion.

The word "percentile" means that the parameter ADC is the value of the apparent diffusion coefficient values that appears at a predetermined place in the set of arranged apparent diffusion coefficient values.

For example, the tenth percentile of the apparent diffusion coefficient values is the value of apparent diffusion coefficient that is such that ninety percents of the apparent diffusion coefficient values are strictly superior to the tenth percentile and ten percents of the diffusion coefficient values are inferior or equal to the tenth percentile.

The second percentile is the value such that ninety-eight percents of the apparent diffusion coefficient values are strictly superior to the second percentile and two percents of the diffusion coefficient values are inferior or equal to the second percentile.

It should be noted that if the number of pixels corresponding to the portion considered is not divisible by 100, the percentile can be calculated by interpolation of the two pixels between which the percentile is comprised.

Unless indicated otherwise, a value "comprised between" two limit values corresponds to a range that includes both limit values.

ADC values are often expressed in square millimeter per second ($mm^2$/s).

When W is a normalized wash-in rate, the first constant $a_1$ is, for example, comprised between $-6 \times 10^{-3}$ $mm^2$ and $-10^{-3}$ $mm^2$. In some embodiments, first constant $a_1$ is comprised between $-5 \times 10^{-3}$ $mm^2$ and $-1.5 \times 10^{-3}$ $mm^2$, for example between $-5 \times 10^{-3}$ $mm^2$ and $-3 \times 10^{-3}$ $mm^2$.

In the present specification, the sign "x" between two numbers indicates multiplication.

Parameter ADC is, notably, a percentile being inferior or equal to 40 when W is a normalized wash-in rate value, in particular a percentile inferior to 30, in particular a percentile inferior to 10.

When W is a normalized wash-out rate, the first constant $a_1$ is, for example, comprised between $2 \times 10^{-2}$ $mm^2$ and

$15 \times 10^{-2}$ mm$^2$.

Parameter ADC is, for example, a percentile being inferior or equal to 15 when W is a normalized wash-out rate value.

The second quantity $x_2$ is calculated, for the first portion considered, according to the equation:

$$x_2 = ADC + b_1 \times TTP \quad \text{(Equation 2)}$$

wherein :

- $b_1$ is a second constant,
- ADC is a percentile, comprised for example between 20 and 45, of the apparent diffusion coefficient values of all pixels corresponding to the first portion, and
- TTP is a time-to-peak value of the first portion.

The second constant $b_1$ is, for example, comprised between $10^{-7}$ and $14 \times 10^{-7}$ mm$^2$/square second (mm$^2$/s$^2$). In some cases, the second constant $b_1$ is comprised between $10^{-7}$ and $9 \times 10^{-7}$ mm$^2$/s$^2$.

The time-to-peak value is a time, on a curve of the values of a T1-weighed signal as a function of time, between a first instant and a second instant. The first instant is a time at which, following injection of the contrast agent to the subject, the values of the T1-weighed signal start to increase (i.e an instant at which the contrast agent injected begins entering the first portion considered). The second instant is a time of maximum concentration of contrast agent in the first portion, corresponding to the maximum intensity of the T1-weighed signal.

The time-to-peak value is, for example, obtained by calculating, for each second image, a mean value of the T1-weighed signals of all pixels, by generating a curve of the means values calculated as a function of time and by measuring the time-to-peak value on the curve.

In a variant, the time-to-peak value is a means value of time-to-peak values calculated for each pixel corresponding to the first portion.

The third quantity $x_3$ is calculated, for the corresponding first portion, according to the equation:

$$x_3 = c_0 + c_1 \times ADC \quad \text{(Equation 3)}$$

wherein :

- $c_0$ is a third constant,
- $c_1$ is a fourth constant, and
- ADC is the percentile inferior or equal to 25 of the apparent diffusion coefficient values of all pixels corresponding to the first portion, for example the tenth percentile or the second percentile.

The third constant $c_0$ is, for example, comprised between 2.41 and 6.44, notably between 3.5 and 5.0. In particular, the third constant $c_0$ is equal to 4.2.

The fourth constant $c_1$ is comprised between -7570 and -4020 s/mm$^2$, notably between -7150 and -5270 seconds/mm$^2$.

The first score P of each first portion is calculated from at least one of the first quantity $x_1$, the second quantity $x_2$ and third quantity $x_3$.

The first score P is, for example, equal to one of the first quantity $x_1$, the second quantity $x_2$ and third quantity $x_3$.

The first score P is, in a possible variant, calculated using a logistic regression.

In statistics, the logistic regression model is used to model the probability of an event existing, for example to model the probability that the first portion contains an aggressive cancer.

The first score P is, for example, calculated using a logistic regression according to the equation:

$$z = e_0 + e_1 x \quad \text{Equation 4}$$

$$P = \frac{e^z}{1 + e^z} \quad \text{Equation 5}$$

wherein x is one of the first quantity $x_1$, the second quantity $x_2$ and third quantity $x_3$, $e_0$ and $e_1$ each being a constant.

In this case, the score P is a probability that the first portion considered contains an aggressive cancer.

For example, in our data, the score $P = \frac{e^z}{1+e^z}$ with x=$x_1$, $a_1$= -2.39x10$^{-3}$, ADC being ADC2 (i.e. second centile of ADC), W being a normalized wash-in rate, $e_0$ = 3.51, e1 = -5.89.10$^3$ was found to be the best logistic regression model for the presence of an ISUP$\geq$2 cancer in the peripheral zone of the prostate.

In another variant, the first score P is obtained by calculating two or three probabilities, each probability being calculated using a logistic regression according to equation 4 for a respective quantity among the first quantity $x_1$, the second quantity $x_2$ and the third quantity $x_3$, and by summing the three calculated probabilities.

It should be noted that, in general, many types of scores P can be envisioned, provided that there is a bijective relationship between the score P calculated and either one of the first quantity $x_1$, the second quantity $x_2$ and third quantity $x_3$ or a combination (such as a sum or a weighted sum) of these quantities.

For example, the score P is obtained by dividing or multiplying one of the first quantity $x_1$, the second quantity $x_2$ and third quantity $x_3$ by a constant, possibly resulting in a dimensionless quantity x, the dimensionless quantity being optionally fed into equation 5 to calculate the score P.

As mentioned previously, the units used to express the parameters ADC, WI, WO and TTP that participate in the calculation of the first quantity $x_1$, the second quantity $x_2$ and third quantity $x_3$ may also be changed, and the parameters a and, $b_1$, adapted accordingly.

Optionally, during the calculation step 130, the control module 15 further calculates a second score Y for the second portion, or each second portion, of the TZ.

The second score Y is calculated as a function of a fourth quantity y. The fourth quantity y is dimensionless.

The fourth quantity y is calculated as a function of the equation:

$$y = d_0 + d_1 \times ADC \qquad \text{(Equation 6)}$$

wherein $d_0$ is a fifth constant, $d_1$ is a sixth constant and ADC is a percentile inferior or equal to 25, notably the tenth percentile, of the apparent diffusion coefficient values in the second portion considered.

The fifth constant $d_0$ is comprised between 2.25 and 13.48, in particular equal to 7.9.

The sixth constant $d_1$ is comprised between -23820 and -4490 s/mm$^2$, notably between -15030 and -6910. In particular, the sixth constant $d_1$ is equal to -10740.

The second score Y is, for example, calculated using the equation:

$$Y = \frac{e^y}{1+e^y} \qquad \text{(Equation 7)}$$

wherein y is the fourth quantity.

However, as is the case for the first score P, many different methods may be used to calculated the second score Y from the fourth quantity y. For example, the second score Y is equal to the fourth quantity y.

During the determination step 140, the control module 15 determines, for each first portion, whether the first portion of the PZ considered comprises an aggressive cancer or not.

In particular, the control module 15 determines whether a first criterion based on the first score P is verified, and determines that the first portion comprises an aggressive cancer if the criterion is verified.

According to an embodiment, the criterion is the fact that the first score P is inferior or equal to a first threshold, in which case the control module compares the first score P to the first threshold, and determines that the first portion comprises an aggressive cancer if the first score P is inferior or equal to the first threshold. If the first score P is strictly superior to the first threshold, the control module 15 determines that the first portion does not comprise an aggressive cancer.

This is notably the case when the first score P is equal to one of the the first quantity $x_1$, the second quantity $x_2$ and third quantity $x_3$. In this case, the first threshold is for example called "score threshold".

The score threshold is, for example, comprised between -0.8x10$^{-3}$ mm$^2$/s and 2.3x10$^{-3}$ mm$^2$/s when the first score P is equal to the first quantity $x_1$, W being a normalized wash-in rate. In particular, the score threshold is comprised between 0.55x10$^{-3}$ mm$^2$/s and 0.8x10$^{-3}$ mm$^2$/s.

When W is a normalized wash-out rate, the score threshold is, for example, comprised between -1.1x10$^{-3}$ and 3.2x10$^{-3}$ mm$^2$/s. In particular, the score threshold is comprised between 0.85 x10$^{-3}$ mm$^2$/s and 1.1x10$^{-3}$ mm$^2$/s.

If the first score P is equal to the second quantity $x_2$, the score threshold is, for example, comprised between -1.1x10$^{-3}$ and 3.3x10$^{-3}$ mm$^2$/s, notably between 0.9 x10$^{-3}$ mm$^2$/s and 1.1x10$^{-3}$ mm$^2$/s.

The score threshold is, for example, equal to 0.40 when the score P is equal to the third quantity $x_3$.

It should be noted that other criteria may be considered.

If the first score P is calculated using equations 4 and 5, the criterion is the fact that the first score P is superior or equal to a first threshold, in which case the control module compares the first score P to the first threshold, and determines that the first portion comprises an aggressive cancer if the first score P is superior or equal to the first threshold. If the first score P is strictly inferior to the first threshold, the control module 15 determines that the first portion does not comprise an aggressive cancer. The first threshold is then, for example, called "probability threshold".

During the determination step 140, the control module 15 further determines, for each second portion, whether the second portion of the TZ considered comprises an aggressive cancer or not.

In particular, the control module 15 checks whether a second criterion is verified, and determines, for each second portion, that the second portion of the TZ considered comprises an aggressive cancer if the criterion is verified. If the criterion is not verified, the control module 15 determines that the second portion does not comprise an aggressive cancer.

According to an embodiment, the control module compares the second score Y to a second threshold, and determines that the second portion comprises an aggressive cancer if the second score Y is inferior or equal to the second threshold. In this case, the criterion is the fact that the second score Y is inferior or equal to the second threshold. However, other criteria may be considered.

For example, the control module 15 calculates a probability that the second portion comprises an aggressive cancer, notably using equation 6, and determines that the second portion comprises an aggressive cancer if the probability is superior or equal to a second probability threshold.

The second probability threshold is, for example, comprised between 0.70 and 0.77 when the value ADC used in equation 6 is a twenty-fifth percentile of ADC.

If the control module 15 has determined that one portion among the first and second portion(s) comprises an aggressive cancer, the control module 15 generates, during the signalling step 150, a message directed to a physician.

The message is intended to be transmitted to the physician by the human-machine interface 20.

The message is a message informing the physician that at least one first or second portion has been determined to contain an aggressive cancer.

The message comprises, for example, an indication of the first or second portion(s) containing an aggressive cancer or aggressive cancers, such as one of the first or second images onto which the first or second portion(s) containing an aggressive cancer or aggressive cancers is (are) delineated in red, or is (are) accompanied by a written message stating that the first or second portion considered is highly likely to be a cancer.

The message may, optionally, be accompanied by a sound indicating the discovery of a cancer, such as an alarm signal.

It should be noted that the message may take any form allowing the information to be understood by the physician.

The physician can then observe in details, on the first or second image(s) or using other methods the first or second portion(s) indicated in the message, in order to determine whether the physician shares the control module's assessment regarding the presence of an aggressive cancer in the first or second portion(s).

In addition, the method enables a physician who has studied the first and second images to have his analysis of the subject's prostate counter-checked by the system 10, which makes it possible to diagnose cancers that have been missed by the physician if the system directs the physician's attention to the possible presence of a cancer in an area of the prostate that may have been overlooked by the physician or in which the cancer does not appear clearly.

In a possible variant, the signalling step 150 is not implemented, and the fact that the control module 15 has determined the presence of an aggressive cancer is, for example, simply stored in the memory 40.

The method for helping diagnose aggressive prostate cancers is, for example, implemented as part of a method for diagnosing prostate cancers, comprising a diagnosis step wherein a prostate cancer is diagnosed as a function of the message(s) generated during the method for helping diagnose aggressive prostate cancers.

In particular, the method for diagnosing prostate cancers is part of a method for treating a prostate cancer, comprising a step for treating a prostate cancer diagnosed by implementing of the method for diagnosing prostate cancers.

The equations 1 to 3 and 5 have been determined by the inventors during the course of a study using a learning database containing MRI sequences from 265 patients who had undergone successively biopsies, multiparametric MRI and a radical prostatectomy because they have been diagnosed and treated for aggressive prostate cancer.

T2-weighted, diffusion-weighted and dynamic contrast-enhanced imaging were systematically recorded and prospectively reviewed by two independent radiologists. On each sequence, they delimited regions suspected to be cancerous. A computer program extracted twenty-three quantitative parameters from these regions.

The learning database contained MR images acquired by several different MR scanners of different manufacturers and types, using different values of magnetic field:

- A Siemens Symphony scanner (1.5 Tesla, 63 patients, 233 suspicious lesions),
- A General Electric Medical Systems Discovery MR 750 scanner (3 T, 124 patients, 272 lesions),
- A Philips medical systems Achieve scanner (3 T, 52 patients, 173 lesions), and
- A Philips healthcare Ingenia scanner (3 T, 26 patients, 56 lesions)

Models were found by machine learning techniques, browsing recursively through the different variables and testing the performance including them one by one. A new variable was retained if the corresponding coefficient and the deviance it explained were statistically significant in the model and if its correlation to each of the other variables in the model was lower than 50%.

The threshold values were determined using an intermediate database containing MR images of prostates containing suspicious lesions, the benign or malignant nature of which had been established by biopsies, and chosen so as to obtain a sensitivity of 90 %.

The intermediate database used MR data obtained from the same GE MR750 and Philips Healthcare Ingenia scanners as the learning database, with data from 101 patients and 11 patients, respectively.

The equations 1 to 3 and 5 were constructed from the selected parameters and their performance was subsequently evaluated on a test database comprising data (MRI images and biopsies) from different patients than the learning and intermediate database. While the learning database comprised only data from patients having confirmed prostate cancer, the test database comprised patients suspicious for prostate cancer, but for which cancer had not been confirmed.

The method for helping diagnosing prostate cancer was implemented on the MRI images of the test database, having two radiologists outline the first and second portions on the MRI images.

A total of 238 suspicious lesions were identified by radiologists on data from 158 patients, with 126 benign lesions (114 in the PZ, 12 in the TZ), 34 cancers having a Gleason score of 6 (30 in the PZ, 4 in the TZ) and 78 cancers having a Gleason score of 7 or more (71 in the PZ, 7 in the TZ), from biopsies.

The test database's MR images were acquired by four different MR scanners:

- A General Electric Medical Systems Discovery MR 750 scanner (3 T, 62 patients, 93 lesions),
- A Philips healthcare Ingenia scanner (3 T, 22 patients, 41 lesions),
- A GE medical systems Optima MR450w (1.5 T, 66 patients, 91 lesions), and
- A Philips Healthcare Ingenia (1.5 T, 8 patients, 13 lesions).

The sensitivity and specificity of systems using each one of equations 1, 2, 3 and 5 were estimated by comparison with diagnosis obtained from biopsies of the corresponding portions of the patient's prostates. The performance of systems was measured from the specificity (called "specificity sp90") that was achieved when sensitivity was set to 90%.

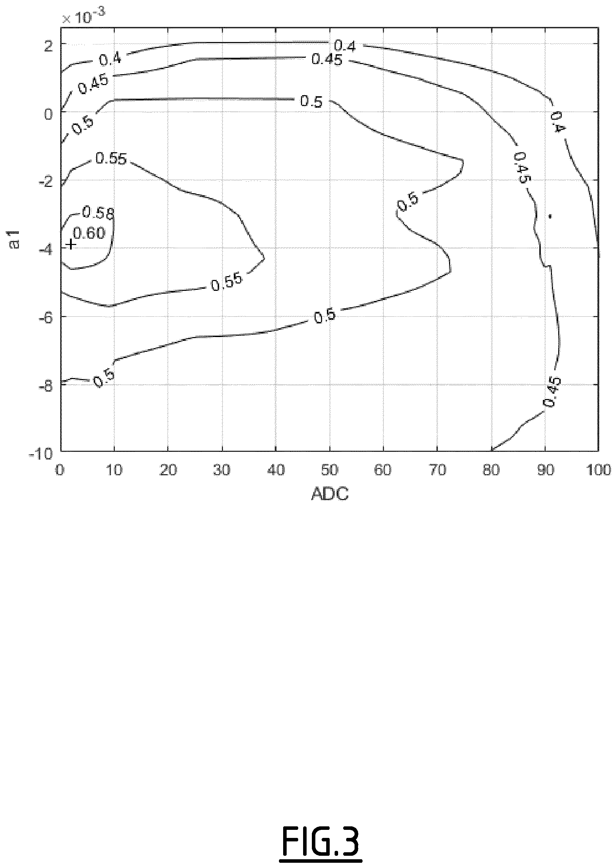

The accuracy of systems 10 using equation 1, with W being a normalized wash-in rate, consistently showed specificities above 0.5 when used on the test database, with ADC being a percentile inferior or equal to 40 and the first constant $a_1$ comprised between $-6 \times 10^{-3}$ and $-10^{-3}$ mm². The values, for a system 10 using equation 1 in the test database, with W being a normalized wash-in rate, of specificity sp90 as a function of ADC and $a_1$ are shown on figure 3.

On figures 3 to 5, the contours delimiting areas of specificity sp90 higher than threshold values are shown, for example the line marked "0.55" contains all areas of the data corresponding to sp90 higher than 0.55.

In particular, when ADC is a percentile inferior or equal to 30 and the first constant $a_1$ comprised between $-5 \times 10^{-3}$ and $-3 \times 10^{-3}$ mm², specificity was systematically over 0.55. When ADC is a percentile inferior or equal to 10 and the first constant $a_1$ comprised between $-4.5 \times 10^{-3}$ and $-3 \times 10^{-3}$ mm², specificity was substantially equal or superior to 0.58.

The best performances were obtained, on the test database, for ADC being the second percentile and the first constant $a_1$ being equal to $-3.96 \times 10^{-5}$ mm², with a specificity equal to 0.606.

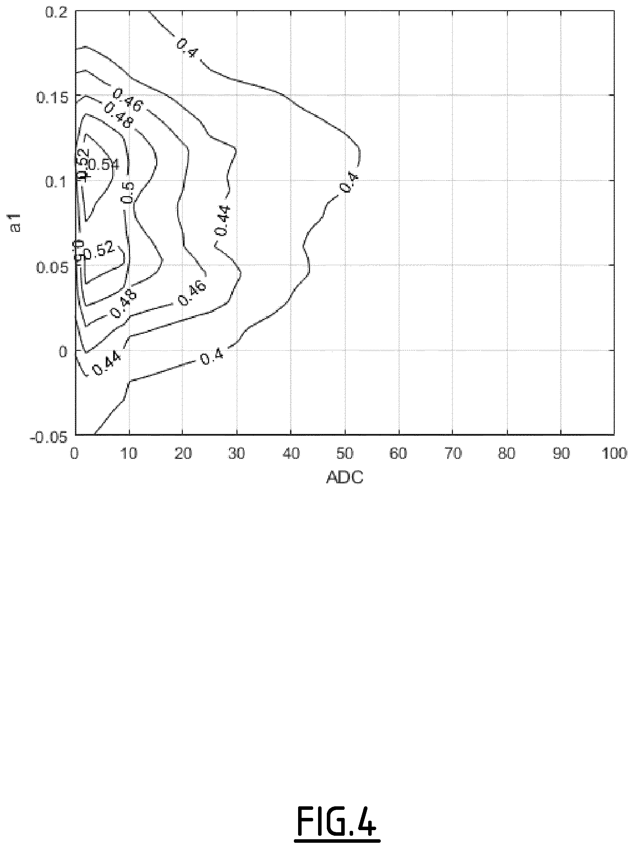

The accuracy of systems 10 using equation 1, with W being a normalized wash-out rate, consistently showed specificities above 0.45 when used on the learning database, with ADC being a percentile inferior or equal to 15 and the second constant $a_1$ comprised between $2 \times 10^{-2}$ and $15 \times 10^{-2}$ mm². The values, for a system 10 using equation 1, in the learning database, with W being a normalized wash-out rate, of specificity sp90 as a function of ADC and $a_1$ are shown on figure 4.

In particular, when ADC is a percentile comprised between 2 and 10 and the first constant $a_1$ comprised between $4 \times 10^{-2}$ and $12 \times 10^{-2}$ mm², specificity was systematically over 0.50.

The best performances were obtained, on the learning database, for ADC being the second percentile and the first constant $a_1$ being equal to $10 \times 10^{-2}$ mm², with a specificity equal to 0.549.

The accuracy of systems 10 using equation 2, consistently showed specificities above 0.45 when used on the test database, with ADC being a percentile comprised between 20 and 45 and the second constant $b_1$ is comprised between $10^{-7}$ and $14 \times 10^{-7}$ mm².

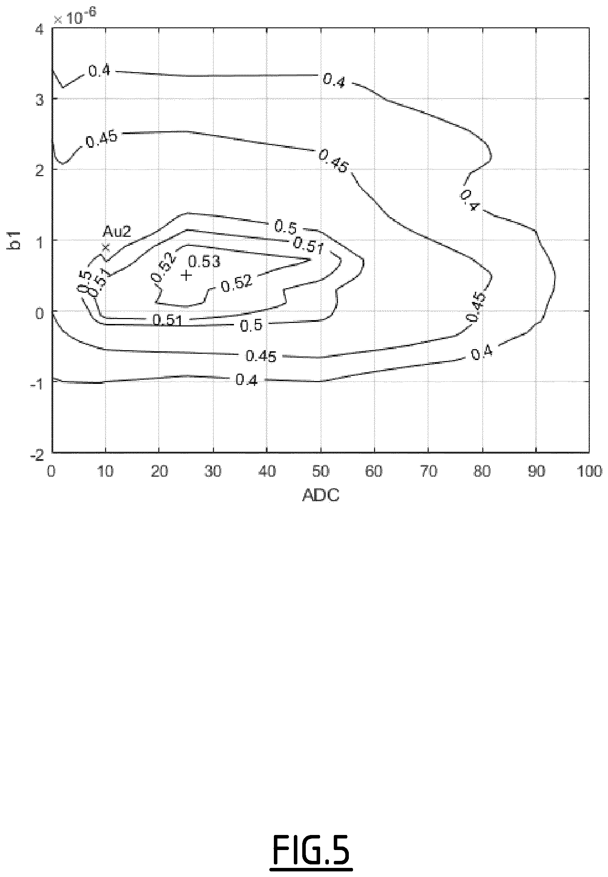

The values, for a system 10 using equation 2, in test learning database, of specificity sp90 as a function of ADC and $b_1$ are shown on figure 5.

In particular, when ADC is a percentile comprised between 20 and 45 and the second constant $b_1$ is comprised between $10^{-7}$ and $9 \times 10^{-7}$ mm², specificity was systematically over 0.50.

The best performances were obtained, on the test database, for ADC being the twenty-fifth percentile and the second constant $b_1$ being equal to $5.1 \times 10^{-7}$ mm², with a specificity equal to 0.539.

Examples of first threshold values and of the constants $c_0$ and $c_1$ determined are listed below, in different cases wherein the system 10 uses equation 4, with the quantity x being the third quantity $x_3$:

a) When ADC is the minimal percentile, the first threshold was comprised between 0.18 and 0.37, the seventh constant $c_0$ was comprised between 2.41 and 3.61, the eight constant $c_1$ was comprised between -5880 and -4020 $s/mm^2$ (the combination giving the best results was: first threshold equal to 0.28, seventh constant $c_0$ equal to 2.9, eight constant $c_1$ equal to -4790 $s/mm^2$);

b) When ADC is the second percentile, the first threshold was comprised between 0.17 and 0.39, the seventh constant $c_0$ was comprised between 3.55 and 4.92, the eight constant $c_1$ was comprised between -7150 and -5270 $s/mm^2$ (the combination giving the best results was: first threshold equal to 0.27, seventh constant $c_0$ equal to 4.17, eight constant $c_1$ equal to -6080 $s/mm^2$);

c) When ADC is the ninth percentile, the first threshold was comprised between 0.17 and 0.38, the seventh constant $c_0$ was comprised between 4.42 and 5.92, the eight constant $c_1$ was comprised between -7570 and -5660 $s/mm^2$ (the combination giving the best results was: first threshold equal to 0.27, seventh constant $c_0$ equal to 5.09, eight constant $c_1$ equal to -6500 $s/mm^2$);

d) When ADC is the tenth percentile, the first threshold was comprised between 0.17 and 0.38, the seventh constant $c_0$ was comprised between 4.47 and 5.96, the eight constant $c_1$ was comprised between -7560 and -5650 $s/mm^2$ (the combination giving the best results was: first threshold equal to 0.26, seventh constant $c_0$ equal to 5.13, eight constant $c_1$ equal to -6490 $s/mm^2$);

e) When ADC is the 25th percentile, the first threshold was comprised between 0.14 and 0.36, the seventh constant $c_0$ was comprised between 4.59 and 6.44, the eight constant $c_1$ was comprised between -7430 and -5260 $s/mm^2$ (the combination giving the best results was: first threshold equal to 0.27, seventh constant $c_0$ equal to 5.49, eight constant $c_1$ equal to -6260 $s/mm^2$).

Examples of second threshold values and of the constants $d_0$ to $d_1$ determined are listed below, in different cases wherein the system 10 uses equation 6:

f) When ADC is the second percentile, the second threshold was comprised between 0.07 and 0.75, the ninth constant $d_0$ was comprised between 2.25 and 13.48, the tenth constant $d_1$ was comprised between -23820 and -4490 $s/mm^2$ (the combination giving the best results was: second threshold equal to 0.4, ninth constant $d_0$ equal to 6.5, tenth constant $d_1$ equal to -11520 $s/mm^2$);

g) When ADC is the ninth percentile, the second threshold was comprised between 0.07 and 0.71, the ninth constant $d_0$ was comprised between 3.83 and 12.5, the tenth constant $d_1$ was comprised between -19500 and -6280 $s/mm^2$ (the combination giving the best results was: second threshold equal to 0.38, ninth constant $d_0$ equal to 7.3, tenth constant $d_1$ equal to -11320 $s/mm^2$);

h) When ADC is the tenth percentile, the second threshold was comprised between 0.06 and 0.71, the ninth constant $d_0$ was comprised between 3.96 and 12.51, the tenth constant $d_1$ was comprised between -19100 and -6430 $s/mm^2$ (the combination giving the best results was: second threshold equal to 0.39, ninth constant $d_0$ equal to 7.4, tenth constant $d_1$ equal to -11360 $s/mm^2$);

i) When ADC is the 25th percentile, the second threshold was comprised between 0.14 and 0.64, the ninth constant $d_0$ was comprised between 4.9 and 11.19, the tenth constant $d_1$ was comprised between -15030 and -6910 $s/mm^2$ (the combination giving the best results was: second threshold equal to 0.4, ninth constant $d_0$ equal to 7.9, tenth constant $d_1$ equal to -10740 $s/mm^2$).

Systems 10 using equation 4, with the quantity x being the first quantity $x_1$, ADC being the second percentile, were found to have the following AUC and specificity when using the outlines of one of the radiologists, (the sensitivity and specificity % when using the outlines of the other radiologist being indicated in parentheses), in the following cases:

- Case a : AUC between 76 % and 86 % (between 78 and 87%), specificity sp90 33 to 52% (28 to 63%),
- Case b : AUC between 77 % and 86 % (between 79 and 87%), specificity sp90 34 to 54% (29 to 66%),
- Case c : AUC between 78 % and 88 % (between 79 and 88%), specificity sp90 37 to 61% (32 to 74%),
- Case d : AUC between 79 % and 88 % (between 79 and 88%), specificity sp90 37 to 62% (32 to 74%),
- Case e : AUC between 79 % and 88 % (between 80 and 89%), specificity sp90 39 to 62% (38 to 72%).

"AUC" is the performance as estimated from the area under a curve of the true positive rate as a function of the false positive rate. Such curves are well-known in the art and often called " Receiver Operating Characteristic (ROC) curves".

Systems 10 using equation 5 were found to have the following AUC and specificity when using the outlines of one of the radiologists, (the sensitivity and specificity % when using the outlines of the other radiologist being indicated in parentheses), in the following cases:

- Case f : AUC between 47 % and 86 % (between 35 and 83%), specificity sp90 0 to 80% (0 to 82%),
- Case g : AUC between 46 % and 88 % (between 28 and 85%), specificity sp90 0 to 83% (0 to 82%),
- Case h : AUC between 46 % and 89 % (between 30 and 85%), specificity sp90 0 to 83% (0 to 82%), and
- Case i : AUC between 50 % and 94 % (between 29 and 82%), specificity sp90 0 to 83% (0 to 75%).

It should be noted that both quantities $x_1$ and $x_2$ combine a centile of ADC and of a dynamic quantity of the MRI images (i.e a quantity that reflects changes as a function of time of a value during inflow or outflow of the contrast agent in or out of the prostate), i.e a wash-in rate, a wash-out rate or a time-to-peak value.

Therefore, part of the invention lies in the discovery, by the inventors, that such combinations are reflective of a likelihood that a cancer is present in a portion of the prostate, and in the discovery of ranges of values $a_1$, $b_1$ and of ADC centiles that, when combined together, lead to an efficient discovery of cancers from the images.

The computer aided diagnosis method described herein may be used to establish a clinical diagnosis based on the results of said method for helping diagnosing aggressive prostate cancers.

In some embodiments, the methods of the present invention are performed in vitro or ex vivo.

Additional validation studies have been performed by the inventors to confirm the initial results of the method. These results are provided below solely as an example.

In this example, the evaluation was performed on another test dataset (named 'external test dataset') independent from the test dataset described above.

This new database is composed of 104 patients with 126 lesions imaged on one scanner, a GE medical systems Signa Voyager scanner (1.5 T, 104 patients, 126 lesions).

These 104 patients have suspected cancer and underwent mpMRI and subsequent biopsy in another institution.

As in the previously described test, the PI-RADSv2 scores (Prostate Imaging-Reporting and Data System version 2) prospectively assigned to each lesion at the time of biopsy were retrieved from the patients' medical records. Then, based on mpMRI reports, two radiologists, with 19 (OR, R1) and 5 (PCM, R2) years of experience, blinded to each other and to biopsy findings, retrospectively outlined the targeted lesions on T2-weighted, diffusion-weighted and DCE images, on the slice level they considered the most representative. The calculation of the lesions' score was performed using the PZ or the TZ model, depending on their location. Thus, each lesion received a PI-RADSv2 score (prospectively assigned at the time of biopsy) and two CAD scores using the regions of interest (ROI) delineated by R1 and R2. The PI-RADSv2 scores and the scores obtained through the invention were then compared to biopsy findings.

The table below details the distribution of the PI-RADSv2 scores and the invention-derived scores as a function of biopsy results.

| | PI-RADSv2 score | CAD result | Biopsy findings | | | | Total |
|---|---|---|---|---|---|---|---|
| | | | Benign | ISUP 1 | ISUP 2 | ISUP $\geq$3 | |
| ROI delineations by practitioner R1 | 1 | CAD - | 8 | 0 | 0 | 0 | 8 |
| | | CAD + | 0 | 0 | 0 | 0 | 0 |
| | 2 | CAD - | 3 | 1 | 0 | 0 | 4 |
| | | CAD + | 0 | 0 | 0 | 0 | 0 |
| | 3 | CAD - | 8 | 1 | 0 | 0 | 9 |
| | | CAD + | 1 | 3 | 0 | 0 | 4 |
| | 4 | CAD - | 18 | 9 | 0 | 2 | 29 |
| | | CAD + | 2 | 12 | 6 | 1 | 21 |
| | 5 | CAD - | 3 | 0 | 0 | 1 | 4 |
| | | CAD + | 1 | 3 | 8 | 13 | 25 |
| | Total | | 44 | 29 | 14 | 17 | 104 |
| | 1 | CAD - | 8 | 0 | 0 | 0 | 8 |
| | | CAD + | 0 | 0 | 0 | 0 | 0 |

(continued)

| | PI-RADSv2 score | CAD result | Biopsy findings | | | | Total |
|---|---|---|---|---|---|---|---|
| | | | Benign | ISUP 1 | ISUP 2 | ISUP ≥3 | |
| ROI delineations by practitioner R2 | 2 | CAD - | 3 | 1 | 0 | 0 | 4 |
| | | CAD + | 0 | 0 | 0 | 0 | 0 |
| | 3 | CAD - | 9 | 3 | 0 | 0 | 12 |
| | | CAD + | 0 | 1 | 0 | 0 | 1 |
| | 4 | CAD - | 18 | 10 | 0 | 2 | 30 |
| | | CAD + | 2 | 11 | 6 | 1 | 20 |
| | 5 | CAD - | 4 | 1 | 0 | 1 | 6 |
| | | CAD + | 0 | 2 | 8 | 13 | 23 |
| | Total | | 44 | 29 | 14 | 17 | 104 |

In the table above, data indicate numbers of patients. ISUP is the abbreviation of the International Society of Urological Pathology grade measuring the severity of a cancer. CAD is the abbreviation of the computer-aided diagnosis system according to the invention.

In the new test dataset, the score AUCs were 82% (95% CI: 76-89) and 86% (95% CI: 79-93) using the ROIs delineated by R1 and R2 respectively. They were not significantly different from that of the PI-RADSv2 score assigned at the time of biopsy (85%, 95% CI: 79-91, p=0.82 and p=1 respectively).

This new test was challenging since all the patients were imaged a scanner different and more recent than those used in the training dataset. Despite this, the system according to the invention provided robust results, not only in terms of overall diagnostic performance (as quantified by the AUC), but also in terms of diagnostic thresholds (as quantified by sensitivity and specificity). Remarkably, the thresholds that provided a 90% sensitivity in the pre-test dataset provided similar sensitivities in the internal (85-89%) and external (92%) test datasets. At this level of sensitivity, the specificity of the computer-aided diagnosis system according to the invention was good (64-76%) suggesting that it could offer a better sensitivity/specificity trade-off than the PI-RADSv2 classical thresholds.

## Claims

1. A system (10) for helping diagnosing aggressive prostate cancers, the system comprising a control module (15), the control module (15) being configured to:

 - receive at least one magnetic resonance image of at least a first portion of the peripheral zone of a subject's prostate, the image comprising a set of pixels, the image comprising, for each pixel, an apparent diffusion coefficient value of a corresponding area of the prostate,
 - calculate a first score, and
 - determine that the first portion contains an aggressive cancer if a first criterion based on the calculated first score is verified, or that the first portion does not contain an aggressive cancer if the first criterion is not verified,

 **characterised by**:
 the first score being calculated as a function of at least one of the following quantities $x_1$ and $x_2$ :

 -

$$x_1 = ADC + a_1 \times W,$$

 wherein W is a value of a normalized wash-in rate or a normalized wash-out rate of the first portion, the wash-in or wash-out value being expressed in Hertz and being estimated from a set of magnetic resonance images of the first portion, ADC being a percentile of the apparent diffusion coefficient values of the first portion and being expressed in units of $mm^2/s$, $a_1$ being a first constant, the first constant being comprised between $-6x10^{-3}$ and $-10^{-3}$ $mm^2$ and

the percentile being inferior or equal to 40 when W is a normalized wash-in rate value, the first constant being comprised between $2\times10^{-2}$ and $15\times10^{-2}$ $mm^2$ and the percentile being inferior or equal to 15 when W is a normalized wash-out rate value,

-

$$x_2 = ADC + b_1 \times TTP,$$

wherein $b_1$ is a second constant comprised between $10^{-7}$ and $14\times10^{-7}$ $mm^2/s^2$, ADC is a percentile comprised between 20 and 45 of the apparent diffusion coefficient values of all pixels corresponding to the first portion, and TTP is a time-to-peak value of the first portion, expressed in seconds, the time-to-peak value being a time duration from a first instant to a second moment, the first instant being a time of arrival of a contrast agent in the prostate, the second instant being a time of maximum contrast concentration of an MRI signal of the first portion.

2. The system according to claim 1, wherein W is a normalized wash-in rate and the first constant $a_1$ is comprised between $-5\times10^{-3}$ and $-3\times10^{-3}$ $mm^2$.

3. The system according to claim 2, wherein the percentile is comprised between 0 and 30.

4. The system according to any one of claims 1 to 3, wherein the second constant $b_1$ is comprised between $10^{-7}$ and $9\times10^{-7}$ $mm^2$/square second.

5. The system according to any one of claims 1 to 4, wherein ADC is the twenty-fifth percentile of the apparent diffusion coefficient values of all pixels corresponding to the first portion when the first score is calculated as a function of the time-to-peak value.

6. The system according to any one of claims 1 to 5, wherein a wash-in or wash-out value is estimated for each pixel corresponding to the first portion, W being a mean value of the wash-in or wash-out values estimated for the pixels.

7. The system according to any one of claims 1 to 5, wherein a plurality of images of the first portion are acquired during a time period, the time period being a time period wherein a contrast agent enters the prostate or a time period wherein a contrast agent leaves the prostate, each image comprising, for each pixel, an intensity value of a T1-weighed signal of the corresponding area of the prostate, the control module being configured to calculate, for each image, a mean value of normalized intensity values of the pixels corresponding to the first portion, each normalized value being equal to the measured value divided by the value of the same pixel before the contrast agent enters the prostate, the normalized wash-in rate value being an ascending slope of a curve of the calculated mean values as a function of time, and the normalized wash-out rate being a descending slope of the curve.

8. The system according to any one of the preceding claims, wherein the control module (15) is configured to compare the score with a threshold and to determine that the first portion comprises an aggressive cancer if the first quantity is inferior or equal to the threshold.

9. The system according to claim 8, wherein the score is equal to the first quantity $x_1$, the threshold being comprised between $-0.8\times10^{-3}$ and $2.3\times10^{-3}$ square millimetres per second when W is a normalized wash-in rate or the threshold being comprised between $-1.1\times10^{-3}$ and $3.2\times10^{-3}$ square millimetres per second when W is a normalized wash-out rate.

10. The system according to claim 8, wherein the score is equal to the second quantity $x_2$, the threshold being comprised between $-1.1\times10^{-3}$ and $3.3\times10^{-3}$ square millimetres per second.

11. The system according to any one of the preceding claims, wherein each image comprises an image of a second portion of the transition zone of the prostate, the control module (15) being further configured to :

- calculate a second score Y as a function of a fourth quantity y, the fourth quantity y being according to the equation:

$$y = d_0 + d_1 \times ADC$$

wherein $d_0$ is a fifth constant, $d_1$ is a sixth constant comprised between -23820 and -4490 seconds/mm$^2$, the fifth constant being notably comprised between 2.25 and 13.48, and ADC is a percentile inferior or equal to 25 of the apparent diffusion coefficient values of all pixels corresponding to the second portion, and
- determine that the second portion comprises an aggressive cancer if a second criterion based on the calculated second score Y is verified, or that the second portion does not contain an aggressive cancer if the first criterion is not verified.

12. A method for helping diagnosing aggressive prostate cancers, the method being implemented by a system (10) comprising a control module (15), the method being implemented by the control module (15) and comprising steps for:

- receiving (110) from a database or a server at least one magnetic resonance image of a first portion of the peripheral zone of a subject's prostate, the image comprising a set of pixels, the image comprising, for each pixel, an apparent diffusion coefficient value of a corresponding area of the first portion,
- calculating (130) a first score, and
- determining (140) that the first portion comprises an aggressive cancer based on the calculated first score,

**characterised by**:
the first score being calculated by the control module (15) as a function of at least one of the following quantities $x_1$ and $x_2$ :

-

$$x_1 = ADC + a_1 \times W,$$

wherein W is a value of a normalized wash-in rate or a normalized wash-out rate of the first portion, the wash-in or wash-out value being expressed in Hertz and being estimated from a set of magnetic resonance images of the first portion, ADC being a percentile of the apparent diffusion coefficient values of the first portion and being expressed in units of mm$^2$/s, $a_1$ being a first constant, the first constant being comprised between $-6 \times 10^{-3}$ and $-10^{-3}$ mm$^2$ and the percentile being inferior or equal to 40 when W is a normalized wash-in rate value, the first constant being comprised between $2 \times 10^{-2}$ and $15 \times 10^{-2}$ mm$^2$ and the percentile being inferior or equal to 15 when W is a normalized wash-out rate value,

-

$$x_2 = ADC + b_1 \times TTP,$$

wherein $b_1$ is a second constant comprised between $10^{-7}$ and $14 \times 10^{-7}$ mm$^2$/s$^2$, ADC is a percentile comprised between 20 and 45 of the apparent diffusion coefficient values of all pixels corresponding to the first portion, and TTP is a time-to-peak value of the first portion, expressed in seconds, the time-to-peak value being a time duration from a first instant to a second moment, the first instant being a time of arrival of a contrast agent in the prostate, the second instant being a time of maximum contrast concentration of an MRI signal of the first portion.

13. A computer program product (45) containing software instructions configured to implement a method according to claim 12 when the software instructions are executed on a processor (35).

14. An information medium (50) containing software instructions configured to implement a method according to claim 12 when the software instructions are executed on a processor (35).

15. An in vitro or ex vivo diagnosis method, comprising: using the method for helping diagnosing aggressive prostate cancers according to claim 12, and establishing a clinical diagnosis based on the results of said method for helping diagnosing aggressive prostate cancers.

**Patentansprüche**

1. System (10) zum Unterstützen einer Diagnose von aggressivem Prostatakrebs, das System umfassend ein Steuermodul (15), wobei das Steuermodul (15) zu Folgendem konfiguriert ist:

- Empfangen mindestens eines Magnetresonanzbilds von mindestens einem ersten Abschnitt der peripheren Zone der Prostata einer Person, das Bild umfassend einen Satz von Pixeln, das Bild umfassend für jedes Pixel einen scheinbaren Diffusionskoeffizientwert eines entsprechenden Bereichs der Prostata,
- Berechnen eines ersten Punktwerts, und
- Bestimmen, dass der erste Abschnitt einen aggressiven Krebs enthält, wenn ein erstes Kriterium basierend auf dem berechneten ersten Punktwert überprüft wird, oder dass der erste Abschnitt keinen aggressiven Krebs enthält, wenn das erste Kriterium nicht überprüft wird,

**dadurch gekennzeichnet, dass**:

der erste Punktwert abhängig von mindestens einer der folgenden Größen $x_1$ und $x_2$ berechnet wird:

-

$$x_1 = ADC + a_1 \times W,$$

wobei W ein Wert einer normalisierten Einwaschrate oder einer normalisierten Auswaschrate des ersten Abschnitts ist, wobei der Einwasch- oder Auswaschwert in Hertz ausgedrückt wird und anhand eines Satzes von Magnetresonanzbildern des ersten Abschnitts geschätzt wird, ADC ein Perzentil der scheinbaren Diffusionskoeffizientwerte des ersten Abschnitts ist und in Einheiten von $mm^2/s$ ausgedrückt wird, $a_1$ eine erste Konstante ist, wobei die erste Konstante zwischen $-6 \times 10^{-3}$ und $-10^{-3}$ $mm^2$ liegt, und das Perzentil kleiner als oder gleich wie 40 ist, wenn W ein normalisierter Wert der Einwaschrate ist, wobei die erste Konstante zwischen $2 \times 10^{-2}$ und $15 \times 10^{-2}$ $mm^2$ liegt, und das Perzentil kleiner als oder gleich wie 15 ist, wenn W ein normalisierter Wert für Auswaschrate ist,

-

$$x_2 = ADC + b_1 \times TTP,$$

wobei $b_1$ eine zweite Konstante ist, die zwischen $10^{-7}$ and $14 \times 10^{-7}$ $mm^2/s^2$ liegt, ADC ein Perzentil zwischen 20 und 45 der scheinbaren Diffusionskoeffizientwerte aller Pixel ist, die dem ersten Abschnitt entsprechen, und TTP ein Zeit-zu-Spitzenwert des ersten Abschnitts ist, ausgedrückt in Sekunden, wobei der Zeit-zu-Spitzenwert eine Zeitdauer von einem ersten Zeitpunkt bis zu einem zweiten Zeitpunkt ist, wobei der erste Zeitpunkt ein Zeitpunkt des Eintreffens eines Kontrastmittels in der Prostata ist, wobei der zweite Zeitpunkt ein Zeitpunkt der maximalen Kontrastkonzentration eines MRI-Signals des ersten Abschnitts ist.

2. System nach Anspruch 1, wobei W eine normalisierte Einwaschrate ist und die erste Konstante $a_1$ zwischen $-5 \times 10^{-3}$ und $-3 \times 10^{-3}$ $mm^2$ liegt.

3. System nach Anspruch 2, wobei das Perzentil zwischen 0 und 30 liegt.

4. System nach einem der Ansprüche 1 bis 3, wobei die zweite Konstante $b_1$ zwischen $10^{-7}$ und $9 \times 10^{-7}$ $mm^2$/Quadratse-kunde liegt.

5. System nach einem der Ansprüche 1 bis 4, wobei ADC das fünfundzwanzigste Perzentil der scheinbaren Diffusionskoeffizientwerte aller Pixel ist, die dem ersten Abschnitt entsprechen, wenn der erste Punktwert abhängig von Zeit-zu-Spitzenwert berechnet wird.

6. System nach einem der Ansprüche 1 bis 5, wobei ein Ein- oder Auswaschwert für jedes Pixel, das dem ersten Abschnitt entspricht, geschätzt wird, wobei W ein Mittelwert der für die Pixel geschätzten Ein- oder Auswaschwerte ist.

7. System nach einem der Ansprüche 1 bis 5, wobei eine Vielzahl von Bildern des ersten Abschnitts während eines Zeitraums erfasst wird, wobei der Zeitraum ein Zeitraum ist, in dem ein Kontrastmittel in die Prostata eintritt oder ein Zeitraum, in dem ein Kontrastmittel die Prostata verlässt, jedes Bild umfassend, für jedes Pixel, einen Intensitätswert eines T1-gewichteten Signals des entsprechenden Bereichs der Prostata, wobei das Steuermodul konfiguriert ist, um für jedes Bild einen Mittelwert der normalisierten Intensitätswerte der dem ersten Abschnitt entsprechenden Pixel zu berechnen, wobei jeder normalisierte Wert gleich wie der gemessene Wert geteilt durch den Wert desselben Pixels vor Eintreten des Kontrastmittels in die Prostata ist, der normalisierte Wert der Einwaschrate eine ansteigende Steigung einer Kurve der berechneten Mittelwerte abhängig von der Zeit ist, und die normalisierte Auswaschrate ein abfallender Kurvenverlauf ist.

8. System nach einem der vorherigen Ansprüche, wobei das Steuermodul (15) konfiguriert ist, um den Punktwert mit einem Schwellenwert zu vergleichen und zu bestimmen, dass der erste Abschnitt einen aggressiven Krebs umfasst, wenn die erste Menge kleiner als oder gleich wie der Schwellenwert ist.

9. System nach Anspruch 8, wobei der Punktwert gleich wie die erste Größe $x_1$ ist, wobei der Schwellenwert zwischen $-0{,}8 \times 10^{-3}$ und $2{,}3 \times 10^{-3}$ Quadratmillimetern pro Sekunde ist, wenn W eine normalisierte Einwaschrate ist, oder der Schwellenwert zwischen $-1{,}1 \times 10^{-3}$ und $3{,}2 \times 10^{-3}$ Quadratmillimetern pro Sekunde liegt, wenn W eine normalisierte Auswaschrate ist.

10. System nach Anspruch 8, wobei der Punktwert gleich wie die zweite Größe $x_2$ ist, wobei der Schwellenwert zwischen $-1{,}1 \times 10^{-3}$ und $3{,}3 \times 10^{-3}$ Quadratmillimetern pro Sekunde liegt.

11. System nach einem der vorherigen Ansprüche, wobei jedes Bild ein Bild eines zweiten Abschnitts des Übergangsbereichs der Prostata umfasst, wobei das Steuermodul (15) ferner zu Folgendem konfiguriert ist:

- Berechnung eines zweiten Punktwerts Y abhängig von einer vierten Größe y, wobei die vierte Größe y der folgenden Gleichung entspricht:

$$y = d_0 + d_1 \times ADC$$

wobei $d_0$ eine fünfte Konstante ist, $d_1$ eine sechste Konstante ist, die zwischen -23820 und -4490 Sekunden/mm$^2$ liegt, wobei die fünfte Konstante insbesondere zwischen 2,25 und 13,48 liegt, und ADC ein Perzentil kleiner als oder gleich wie 25 der Werte des scheinbaren Diffusionskoeffizienten aller dem zweiten Teil entsprechenden Pixel ist, und
- Bestimmen, dass der zweite Abschnitt einen aggressiven Krebs enthält, wenn ein zweites Kriterium basierend auf der berechneten zweiten Punktwert Y überprüft wird, oder dass der zweite Teil keinen aggressiven Krebs enthält, wenn das erste Kriterium nicht überprüft wird.

12. Verfahren zum Unterstützen einer Diagnose von aggressivem Prostatakrebs, wobei das Verfahren durch ein System (10) implementiert wird, umfassend ein Steuermodul (15), wobei das Verfahren durch das Steuermodul (15) implementiert wird und Schritte zu Folgendem umfasst:

- Empfangen (110), von einer Datenbank oder einem Server,
mindestens eines Magnetresonanzbilds eines ersten Abschnitts der peripheren Zone der Prostata einer Person, das Bild umfassend einen Satz von Pixeln, wobei das Bild für jedes Pixel einen scheinbaren Diffusionskoeffizientwert eines entsprechenden Bereichs des ersten Abschnitts umfasst,
- Berechnen (130) eines ersten Punktwerts, und
- Bestimmen (140), dass der erste Abschnitt einen aggressiven Krebs umfasst, basierend auf dem berechneten ersten Punktwert,

**dadurch gekennzeichnet, dass**:
der erste Punktwert abhängig von mindestens einer der folgenden Größen $x_1$ und $x_2$ durch das Steuermodul (15) berechnet wird:

-

$$x_1 = ADC + a_1 \times W,$$

wobei W ein Wert einer normalisierten Einwaschrate oder einer normalisierten Auswaschrate des ersten Abschnitts ist, wobei der Einwasch- oder Auswaschwert in Hertz ausgedrückt wird und anhand eines Satzes von Magnetresonanzbildern des ersten Abschnitts geschätzt wird, ADC ein Perzentil der scheinbaren Diffusionskoeffizientwerte des ersten Abschnitts ist und in Einheiten von mm$^2$/s ausgedrückt wird, $a_1$ eine erste Konstante ist, wobei die erste Konstante zwischen $-6 \times 10^{-3}$ und $-10^{-3}$ mm$^2$ liegt, und das Perzentil kleiner als oder gleich wie 40 ist, wenn W ein normalisierter Wert der Einwaschrate ist, wobei die erste Konstante zwischen $2 \times 10^{-2}$ und

15x10$^{-2}$ mm$^2$ liegt, und das Perzentil kleiner als oder gleich wie 15 ist, wenn W ein normalisierter Wert für Auswaschrate ist,

-

$$x_2 = ADC + b_1 \times TTP,$$

wobei b$_1$ eine zweite Konstante ist, die zwischen 10$^{-7}$ und 14x10$^{-7}$ mm$^2$/s$^2$ ist, ADC ein Perzentil zwischen 20 und 45 der scheinbaren Diffusionskoeffizientwerte aller Pixel ist, die dem ersten Abschnitt entsprechen, und TTP ein Zeit-zu-Spitzenwert des ersten Abschnitts ist, ausgedrückt in Sekunden, wobei der Zeit-zu-Spitzenwert eine Zeitdauer von einem ersten Zeitpunkt bis zu einem zweiten Zeitpunkt ist, wobei der erste Zeitpunkt ein Zeitpunkt des Eintreffens eines Kontrastmittels in der Prostata ist, wobei der zweite Zeitpunkt ein Zeitpunkt der maximalen Kontrastkonzentration eines MRI-Signals des ersten Abschnitts ist.

13. Computerprogrammprodukt (45), das Softwareanweisungen enthält, die konfiguriert sind, um ein Verfahren nach Anspruch 12 zu implementieren, wenn die Softwareanweisungen auf einem Prozessor (35) ausgeführt werden.

14. Informationsträger (50), der Softwareanweisungen enthält, die konfiguriert sind, dass sie ein Verfahren nach Anspruch 12 zu implementieren, wenn die Softwareanweisungen auf einem Prozessor (35) ausgeführt werden.

15. In-vitro- oder ex-vivo-Diagnoseverfahren, umfassend: Verwenden des Verfahrens zum Unterstützen einer Diagnose von aggressivem Prostatakrebs nach Anspruch 12 und Erstellen einer klinischen Diagnose basierend auf den Resultaten des Verfahrens zum Unterstützen einer Diagnose von aggressivem Prostatakrebs.

**Revendications**

1. Système (10) d'aide au diagnostic des cancers agressifs de la prostate, le système comprenant un module de contrôle (15), le module de contrôle (15) étant configuré pour :

   - recevoir au moins une image par résonance magnétique d'au moins une première partie de la zone périphérique de la prostate d'un sujet, l'image comprenant un ensemble de pixels, l'image comprenant, pour chaque pixel, une valeur de coefficient de diffusion apparente d'une zone correspondante de la prostate,
   - calculer un premier score, et
   - déterminer que la première partie contient un cancer agressif si un premier critère basé sur le premier score calculé est vérifié, ou que la première partie ne contient pas de cancer agressif si le premier critère n'est pas vérifié,

   **caractérisé par** :
   le premier score est calculé en fonction d'au moins une des quantités suivantes x$_1$ et x$_2$ :

   -

$$x_1 = ADC + a_1 \times W,$$

   où W est une valeur d'un taux de captation normalisé ou d'un taux d'élimination normalisé de la première partie, la valeur de captation ou d'élimination étant exprimée en Hertz et étant estimée à partir d'un ensemble d'images par résonance magnétique de la première partie, ADC étant un centile des valeurs du coefficient de diffusion apparente de la première partie et étant exprimée en unités de mm$^2$/s, a$_1$ étant une première constante, la première constante étant comprise entre -6x10$^{-3}$ et -10$^{-3}$ mm$^2$ et le centile étant inférieur ou égal à 40 lorsque W est une valeur de taux de captation normalisé, la première constante étant comprise entre 2x10$^{-2}$ et 15x10$^{-2}$ mm$^2$ et le centile étant inférieur ou égal à 15 lorsque W est une valeur de taux d'élimination normalisé,

   -

$$x_2 = ADC + b_1 \times TTP,$$

   où b$_1$ est une seconde constante comprise entre 10$^{-7}$ et 14x10$^{-7}$ mm$^2$/s$^2$ADC est un centile compris entre 20 et 45 des valeurs du coefficient de diffusion apparente de tous les pixels correspondant à la première partie, et TTP est une valeur de temps de montée-crête de la première partie, exprimée en secondes, la valeur de temps de montée-crête étant une durée de temps entre un premier instant et un second instant, le premier instant étant un temps d'arrivée d'un agent de contraste dans la prostate, le second instant étant un temps de concentration maximale de contraste d'un signal IRM de la première partie.

2. Système selon la revendication 1, dans lequel W est un taux de captation normalisé et la première constante $a_1$ est comprise entre $-5 \times 10^{-3}$ et $-3 \times 10^{-3}$ mm$^2$.

3. Système selon la revendication 2, dans lequel le centile est compris entre 0 et 30.

4. Système selon l'une quelconque des revendications 1 à 3, dans lequel la seconde constante $b_1$ est comprise entre $10^{-7}$ et $9 \times 10^{-7}$ mm$^2$/seconde carrée.

5. Système selon l'une quelconque des revendications 1 à 4, dans lequel ADC est le vingt-cinquième centile des valeurs du coefficient de diffusion apparente de tous les pixels correspondant à la première partie lorsque le premier score est calculé en fonction de la valeur de temps de montée-crête.

6. Système selon l'une quelconque des revendications 1 à 5, dans lequel une valeur de captation ou d'élimination est estimée pour chaque pixel correspondant à la première partie, W étant une valeur moyenne des valeurs de captation ou d'élimination estimées pour les pixels.

7. Système selon l'une quelconque des revendications 1 à 5, dans lequel plusieurs images de la première partie sont acquises pendant une période de temps, la période de temps étant une période de temps pendant laquelle un agent de contraste pénètre dans la prostate ou une période de temps pendant laquelle un agent de contraste quitte la prostate, chaque image comprenant, pour chaque pixel, une valeur d'intensité d'un signal pondéré T1 de la zone correspondante de la prostate, le module de contrôle étant configuré pour calculer, pour chaque image, une valeur moyenne des valeurs d'intensité normalisées des pixels correspondant à la première partie, chaque valeur normalisée étant égale à la valeur mesurée divisée par la valeur du même pixel avant que l'agent de contraste ne pénètre dans la prostate, la valeur du taux de captation normalisé étant une pente ascendante d'une courbe des valeurs moyennes calculées en fonction du temps, et le taux d'élimination normalisé étant une pente descendante de la courbe.

8. Système selon l'une quelconque des revendications précédentes, dans lequel le module de contrôle (15) est configuré pour comparer le score à un seuil et pour déterminer que la première partie comprend un cancer agressif si la première quantité est inférieure ou égale au seuil.

9. Système selon la revendication 8, dans lequel le score est égal à la première quantité $x_1$, le seuil étant compris entre $-0,8 \times 10^{-3}$ et $2,3 \times 10^{-3}$ millimètres carrés par seconde lorsque W est un taux de captation normalisé ou le seuil étant compris entre $-1,1 \times 10^{-3}$ et $3,2 \times 10^{-3}$ millimètres carrés par seconde lorsque W est un taux d'élimination normalisé.

10. Système selon la revendication 8, dans lequel le score est égal à la deuxième quantité $x_2$ le seuil étant compris entre $-1,1 \times 10^{-3}$ et $3,3 \times 10^{-3}$ millimètres carrés par seconde.

11. Système selon l'une quelconque des revendications précédentes, dans lequel chaque image comprend une image d'une deuxième partie de la zone de transition de la prostate, le module de contrôle (15) étant en outre configuré pour :

- calculer un deuxième score Y en fonction d'une quatrième quantité y, la quatrième quantité y étant conforme à l'équation :

$$y = d_0 + d_1 \times ADC$$

où $d_0$ est une cinquième constante, $d_1$ est une sixième constante comprise entre -23820 et -4490 secondes/mm$^2$, la cinquième constante étant notamment comprise entre 2,25 et 13,48, et ADC est un centile inférieur ou égal à 25 des valeurs du coefficient de diffusion apparente de tous les pixels correspondant à la deuxième partie, et
- déterminer que la deuxième partie comprend un cancer agressif si un deuxième critère basé sur le deuxième score Y calculé est vérifié, ou que la deuxième partie ne contient pas de cancer agressif si le premier critère n'est pas vérifié.

12. Procédé d'aide au diagnostic des cancers agressifs de la prostate, le procédé étant mis en œuvre par un système (10) comprenant un module de contrôle (15), le procédé étant mis en œuvre par le module de contrôle (15) et comprenant des étapes pour :

- recevoir (110) d'une base de données ou d'un serveur au moins une image par résonance magnétique d'une première partie de la zone périphérique de la prostate d'un sujet, l'image comprenant un ensemble de pixels, l'image comprenant, pour chaque pixel, une valeur de coefficient de diffusion apparent d'une zone correspondante de la première partie,
- calculer (130) un premier score, et
- déterminer (140) que la première partie comprend un cancer agressif sur la base du premier score calculé,

**caractérisé par** :
le premier score est calculé par le module de contrôle (15) en fonction d'au moins une des quantités suivantes $x_1$ et $x_2$ :

-

$$x_1 = ADC + a_1 \times W,$$

où W est une valeur d'un taux de captation normalisé ou d'un taux d'élimination normalisé de la première partie, la valeur de captation ou d'élimination étant exprimée en Hertz et étant estimée à partir d'un ensemble d'images par résonance magnétique de la première partie, ADC étant un centile des valeurs du coefficient de diffusion apparente de la première partie et étant exprimée en unités de $mm^2/s$, $a_1$ étant une première constante, la première constante étant comprise entre $-6 \times 10^{-3}$ et $-10^{-3}$ $mm^2$ et le centile étant inférieur ou égal à 40 lorsque W est une valeur de taux de captation normalisé, la première constante étant comprise entre $2 \times 10^{-2}$ et $15 \times 10^{-2}$ $mm^2$ et le centile étant inférieur ou égal à 15 lorsque W est une valeur de taux d'élimination normalisé,

-

$$x_2 = ADC + b_1 \times TTP,$$

où $b_1$ est une seconde constante comprise entre $10^{-7}$ et $14 \times 10^{-7}$ $mm^2/s^2$ ADC est un percentile compris entre 20 et 45 des valeurs du coefficient de diffusion apparente de tous les pixels correspondant à la première partie, et TTP est une valeur de temps de montée-crête de la première partie, exprimée en secondes, la valeur de temps de montée-crête étant une durée de temps entre un premier instant et un second instant, le premier instant étant un temps d'arrivée d'un agent de contraste dans la prostate, le second instant étant un temps de concentration maximale de contraste d'un signal IRM de la première partie.

13. Produit de programme informatique (45) contenant des instructions logicielles configurées pour mettre en œuvre un procédé selon la revendication 12 lorsque les instructions logicielles sont exécutées sur un processeur (35).

14. Support d'information (50) contenant des instructions logicielles configurées pour mettre en œuvre un procédé selon la revendication 12 lorsque les instructions logicielles sont exécutées sur un processeur (35).

15. Procédé de diagnostic in vitro ou ex vivo, comprenant : utiliser le procédé d'aide au diagnostic des cancers agressifs de la prostate selon la revendication 12, et établir un diagnostic clinique basé sur les résultats dudit procédé d'aide au diagnostic des cancers agressifs de la prostate.

EP 4 330 910 B1

FIG.1

100

110

120

130

140

150

## FIG.2

## FIG.3

FIG.4

# FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **AU HOANG DINH et al.** Quantitative analysis of prostate multiparametric MR images for detection of aggressive prostate cancer in the peripheral zone : a multiple imager study. *I Radiology*, 2016, vol. 280 (1), 117

- Characterization of prostate cancer with Gleason score of at least 7 by using quantitative multiparametric MR imaging : validation of a computer-aided diagnosis system in patients referred for prostate biopsy. *Radiology*, 2018, vol. 287 (2), 525